# EUROPEAN PATENT SPECIFICATION

(11) **EP 3 687 483 B1**
(45) Date of publication and mention of the grant of the patent: **30.07.2025**
(21) Application number: 18778492.1
(22) Date of filing: 28.09.2018
(51) Int. Cl.: A61K 8/37, A61Q 5/12, A61K 8/81

(54) **COMPOSITION FOR CARING FOR KERATIN FIBRES**
ZUSAMMENSETZUNG ZUR PFLEGE VON KERATINFASERN
COMPOSITION DE SOIN DES FIBRES KERATINIQUES

(30) Priority: 29.09.2017 FR 1759137
(43) Date of publication of application: 05.08.2020
(73) Proprietor: L'OREAL, 75008 Paris (FR)
(72) Inventor: BRUEL, Emilie, 94152 CHEVILLY LA RUE (FR); BOUCHARD, Fabienne, 94152 CHEVILLY LA RUE (FR)
(74) Representative: L'Oreal
(86) International application number: PCT/EP2018/076528
(87) International publication number: WO 2019/063823

(56) References cited:
- DATABASE GNPD [online] MINTEL; 3 March 2011 (2011-03-03), ANONYMOUS: "Lip Gloss", XP55786199, retrieved from https://www.gnpd.com/sinatra/recordpage/1507495/ Database accession no. 1507495
- DATABASE GNPD [online] MINTEL; 1 May 2017 (2017-05-01), "Anti Oxi+ Skin Refining Cleansing Oil", XP002778638, Database accession no. 4820349
- DATABASE GNPD [online] MINTEL; 1 July 2016 (2016-07-01), "The smoothing & Purifying Cleansing Oil", XP002778639, Database accession no. 4146727
- DATABASE GNPD MINTEL; July 2017 (2017-07-01), AVON: "Kiss "n" Go Lipstick", XP002778640, Database accession no. 4975095

## Description

A subject of the invention is a composition the caring for keratin fibres intended for the cosmetic treatment of keratin fibres and in particular the conditioning of keratin fibres, in particular in terms of disentangling, straightening, combability and manageability of keratin fibres. Another subject-matter of the invention is the use of such a composition for conditioning keratin fibres.

It is well known that human keratin fibres, corresponding in particular to the hair, the eyebrows, the eyelashes, beard hairs, moustache hairs or pubic hairs, can be sensitized, in particular damaged and/or embrittled, to various degrees, under the action of atmospheric agents or under the action of mechanical and/or chemical treatments, such as cleansing, styling, cutting, dyeing, bleaching or permanent-waving. These keratin fibres then become difficult to disentangle and to style, and also lack suppleness and softness.

Compositions containing fatty substances as conditioning agents have already been proposed for the treatment of keratin fibres and in particular the hair, the beard or the moustache. However, such compositions generally comprise high contents of plant oils, which have the drawback of developing an unpleasant odour as they become oxidized in contact with the air. It has already been proposed to replace said plant oils with synthetic oils, but the resulting compositions do not yet have the desired cosmetic qualities, in particular in terms of sensory properties, in particular of feel of the keratin fibres after treatment. It in particular appears that the known compositions leave a tacky feeling which detrimentally modifies the feel and/or the styling of these keratin fibres even after drying.

It is therefore of great interest to find compositions for caring for keratin fibres which make it possible to improve the straightening, disentangling, combability and manageability of keratin fibres such as the hair, beard hairs or moustache hairs; to facilitate their shaping and to make the feel of the keratin fibres more pleasant and fluid.

A commercialized product, i.e "The smoothing & purifying cleansing oil", from GNPD (online) Mintel, discloses a cleansing oil comprising ethylhexyl palmitate, isopropyl myristate, polybutene, and caprylic/ capric triglyceride. It does not comprise any silicone compounds and plant oil(s). However, it does not comprise polybutene in the required amount.

This technical problem is solved with the present invention, a subject of which is a composition comprising:
a) a) from 8 to 15% polybutene by weight relative to the total weight of the composition;
b) at least one first non-silicone liquid fatty substance; and
c) at least one second non-silicone liquid fatty substance, different from b),
wherein said composition comprises less than 5% by weight of plant oil, relative to the total weight of the composition, does not contain any silicone compounds, and is anhydrous.

Said at least one first non-silicone liquid fatty substance is chosen from liquid esters of saturated or unsaturated, linear or branched, C₁-C₂₆ aliphatic monoacids and of saturated or unsaturated, linear or branched, C₁-C₂₆ aliphatic monoalcohols, the total number of carbon atoms in the esters being greater than or equal to 10; preferably from monoesters of monoacids and of monoalcohols; and more preferentially from ethyl and isopropyl palmitates. Said first liquid fatty substance is present in a content ranging from 10% to 80% by weight and preferably from 55% to 70% by weight, relative to the total weight of the composition.

In one particular embodiment, said at least one second liquid fatty substance is chosen from natural or synthetic esters of mono-, di- or triacids with glycerol and comprising at least caprylic/capric acid triglycerides. It is present in a total content ranging from 10% to 60% by weight and preferably from 15% to 25% by weight, relative to the total weight of the composition.

In one particular embodiment, the composition according to the invention also comprises at least one liquid polar solvent, preferably at least one alkanol and more preferably ethyl alcohol. Said liquid polar solvent is preferably present in a content ranging from 2% to 20% and preferably from 2.5% to 7.5% by weight, relative to the total weight of the composition.

The composition according to the invention is anhydrous, does not comprise plant oils, and does not contain silicone compounds.

The present invention also relates to the use of a composition according to the invention for conditioning keratin fibres, and in particular in terms of disentangling, straightening, combability and manageability of keratin fibres, and more particularly keratin fibres chosen from the hair, eyelashes, eyebrows, beard hairs, moustache hairs and pubic hairs. Preferably, the composition according to the invention is used for conditioning beard and/or moustache hairs.

The present invention also relates to a process for conditioning keratin fibres, by treatment or application to said keratin fibres of a composition according to the invention.

### DESCRIPTION

For the purposes of the present invention, the term "keratin fibres" is intended to mean in particular human keratin fibres, and more particularly the hair, body hairs, in particular beard and/or moustache hairs, eyelashes, eyebrows and pubic hairs.

### POLYBUTENE

The composition according to the invention comprises polybutene.

The polybutene used in a composition according to the invention is preferably likened to a non-volatile liquid fatty substance.

The term "liquid fatty substance" is intended to mean an organic compound which is water-insoluble at ambient temperature of 25°C and at atmospheric pressure, but which is in liquid form under these temperature and atmospheric pressure conditions. The liquid fatty substances according to the invention preferably have a viscosity of less than or equal to 2 Pa.s, better still less than or equal to 1 Pa.s and even better still less than or equal to 0.1 Pa.s at 25°C and at a shear rate of 1 s-1.

The term "non-volatile liquid fatty substance" is intended to mean a liquid fatty substance which remains on the keratin fibre at ambient temperature and atmospheric pressure. More precisely, a non-volatile liquid fatty substance has an evaporation rate strictly less than 0.01 mg/cm2/min.

To measure this evaporation rate, 15 g of volatile liquid fatty substance or mixture of volatile liquid fatty substances to be tested are placed in a crystallizing dish 7 cm in diameter, placed on a balance that is in a large chamber of about 0.3 m³ which is temperature-regulated, at a temperature of 25°C, and hygrometry-regulated, at a relative humidity of 50%. The liquid is allowed to evaporate freely, without stirring it, while providing ventilation by means of a fan (Papst-Motoren, reference 8550 N, rotating at 2,700 rpm) placed in a vertical position above the crystallizing dish containing said liquid fatty substance or said mixture, the blades being directed towards the crystallizing dish, 20 cm away from the bottom of the crystallizing dish. The weight of of liquid fatty substance remaining in the crystallizing dish is measured at regular intervals. The evaporation rates are expressed in mg of liquid fatty substance evaporated per unit of surface area (cm²) and per unit of time (minutes).

A polybutene according to the invention advantageously contains at least 10 monomers, preferentially between 12 and 50 monomers and even more preferentially between 15 and 40 monomers.

A polybutene according to the invention has a weight-average molecular weight Mw of greater than or equal to 750 g/mol, advantageously between 800 and 10 000 g/mol, more preferentially between 900 and 5 000 g/mol.

Preferably, said polybutene comprises at least one monomer chosen from the four isomers of butene structure, namely 1-butene, (Z)-2-butene, (E)-2-butene and 2-methyl-1-propene (or isobutene), and mixtures thereof. The term "polybutene" covers polymers derived from at least any one of these monomers in the rest of the present description.

Preferably, said polybutene comprises at least one 2-methylprop-1-ene monomer.

According to one particular embodiment, said polybutene is derived from the copolymerization of at least two of these four isomers. Preferably, one of these two isomers is 2-methyl-1-propene. According to a particular embodiment, said resulting polybutene comprises a mixture of poly-1-butene and 2-methyl-1-propene monomers.

Preferably, said polybutene is partially hydrogenated.

The polybutene according to the invention advantageously has at 100°C a kinematic viscosity of greater than 1,000 centistokes, preferably between 2,000 and 8,000 centistokes, more preferentially between 2,500 and 5,000 centistokes.

The polybutene according to the invention has at 25°C a static viscosity of greater than or equal to 20 000 mPa.s, preferably greater than or equal to 65 000 mPa.s, preferably greater than or equal to 350 000 mPa.s, for example inclusively between 25 000 and 800 000 mPa.s and better still between 400 000 and 600 000 mPa.s. Such a viscosity may be measured using an RS75 rheometer sold by the company Haake, equipped with a plate or cone 60 mm in diameter with a 2° incline.

The polybutene used in the context of the present invention may be chosen, for example, from Indopol H-100, Indopol H-300 and Indopol H-1500 from the company Amoco or Ineos, and Parleam V, Parleam HV and Parleam SV from NOF Corporation. Indopol H-1500 from the company Ineos will preferentially be used.

The total content of polybutene in the composition according to the invention ranges from 5% to 30% by weight, in particular from 7% to 20% by weight and preferably from 8% to 15% by weight relative to the total weight of the composition.

### FIRST NON-SILICONE LIQUID FATTY SUBSTANCE

The composition according to the invention also comprises at least one first non-silicone liquid fatty substance chosen from: monoesters of saturated or unsaturated, linear or branched C₁-C₂₆ aliphatic monoacids and of saturated or unsaturated, linear or branched C₁-C₂₆ aliphatic monoalcohols, the total number of carbon atoms of the esters being greater than or equal to 10.

Preferably, for the esters of monoalcohols, at least one from among the alcohol and the acid from which the esters of the invention are derived is branched.

Among the monoesters of monoacids and of monoalcohols, mention may be made of ethyl palmitate, isopropyl palmitate, alkyl myristates such as isopropyl myristate or ethyl myristate, isocetyl stearate, 2-ethylhexyl isononanoate, isononyl isononanoate, isodecyl neopentanoate and isostearyl neopentanoate.

In one particular embodiment, the composition according to the invention comprises from 10% to 80% by weight, preferably from 55% to 70% by weight, of said first non-silicone liquid fatty substance, relative to the total weight of the composition.

### SECOND NON-SILICONE LIQUID FATTY SUBSTANCE

The composition according to the invention also comprises at least one second non-silicone liquid fatty substance different from said at least first non-silicone liquid fatty substance. Said second non-silicone liquid fatty substance for use in the invention is from natural or synthetic origin. In a preferred embodiment, said second non-silicone liquid fatty substance is chosen from natural or synthetic esters of mono-, di-or triacids with glycerol, comprising at least caprylic/capric triglycerides.

In one particular embodiment, the composition according to the invention comprises from 10% to 60% by weight, preferably from 15% to 25% by weight, of the second non-silicone liquid fatty substance, relative to the total weight of the composition.

### LIQUID POLAR ORGANIC SOLVENT

In one particular embodiment, the composition according to the present invention may also comprise at least one polar organic solvent which is liquid at ambient temperature (25°C). The polar solvents that can be used in the composition according to the invention are in particular chosen from alkanols such as ethyl alcohol, isopropyl alcohol, aromatic alcohols such as benzyl alcohol, and phenylethyl alcohol, or polyols or polyol ethers, for instance ethylene glycol monomethyl, monoethyl or monobutyl ether, propylene glycol or ethers thereof, for instance propylene glycol monomethyl ether, butylene glycol, dipropylene glycol, and also diethylene glycol alkyl ethers, for instance diethylene glycol monoethyl ether or monobutyl ether.

If one or more polar organic solvents are present in the composition according to the invention, their respective content in the composition ranges from 2% to 20% and preferably from 2.5% to 7.5% by weight relative to the total weight of the composition.

In one preferred embodiment, the composition according to the invention comprises ethyl alcohol, preferably in a content ranging from 2% to 20%, and more preferably from 2.5% to 7.5% by weight, relative to the total weight of the composition.

The composition according to the invention is anhydrous. The term "anhydrous" is intended to mean a composition according to the invention not containing any added water, that is to say that the water that may be present comes only from the water of crystallization or of adsorption of the starting materials. In any case, a composition according to the invention contains less than 5% by weight of water and preferably less than 1% by weight of water relative to the total weight of the composition.

In one particular embodiment, the composition according to the invention is a non-silicone composition. There is in fact a need to provide non-silicone, for example natural, cosmetic compositions intended for the conditioning of keratin fibres, which make it possible to satisfactorily condition keratin fibres, and which are in particular capable of conferring on them good properties in terms of suppleness, body, straightening, combing and shaping.

For the purposes of the invention, the term "non-silicone composition" is intended to mean a composition containing less than 1% by weight of silicone compound(s), preferably less than 0.5% by weight of silicone compound(s), more preferentially less than 0.3% by weight of silicone compound(s), and better still less than 0.1% by weight of silicone compound(s), relative to the total weight of the composition. Preferably, the composition is totally free of silicone compound(s).

For the purposes of the invention, the term "silicone compound" is intended to mean a compound containing one or more silicone atoms, and preferably a silicone-comprising fatty substance.

The composition according to the invention may also comprise one or more additional non-silicone fatty substances, that is to say of which the structure does not contain any silicone atoms. The additional non-silicone fatty substances thus generally have in their structure a hydrocarbon-based chain comprising at least 6 carbon atoms and not comprising any siloxane groups. The additional non-silicone fatty substances that can be used in the composition according to the invention are generally soluble in organic solvents under the same temperature and pressure conditions, for instance chloroform, ethanol, benzene, liquid petroleum jelly or decamethylcyclopentasiloxane. The additional non-silicone fatty substances that may be used in the composition according to the invention are generally not oxyalkylenated and preferably do not contain any carboxylic acid COOH functions.

Preferably, the additional non-silicone fatty substances are chosen from hydrocarbons, fatty alcohols, fatty esters, fatty ethers, non-silicone waxes and mixtures thereof.

The compositions according to the invention may also comprise one or more additives chosen from fixing polymers, ceramides or, pseudoceramides, vitamins and provitamins, including panthenol, water-soluble or liposoluble, non-silicone sunscreens, nacreous agents and opacifiers, sequestrants, conditioning agents different from those above, solubilizers, antioxidants, antidandruff agents, anti-seborrhoeic agents, keratin-fibre-loss counteractants and/or keratin fibre restorers, penetrants, fragrances, peptizers and preserving agents, essential oils or any other additive conventionally used in the cosmetics field.

These additives may be present in the composition according to the invention in an amount ranging from 0 to 20% by weight, relative to the total weight of the composition.

Those skilled in the art will take care to select these optional additives and the amounts thereof so that they do not harm the properties of the compositions of the present invention.

Needless to say, the composition of the invention should be cosmetically or dermatologically acceptable, i.e., it should contain a non-toxic physiologically acceptable medium and should be able to be applied to the keratin fibers, and more particularly to those of human beings. For the purposes of the invention, the expression "cosmetically acceptable" means a composition of pleasant appearance, odor, feel and/or taste. In a particular embodiment, compositions according the invention are cosmetic and/or non-therapeutic compositions.

### EXAMPLES

### Example 1

The following compositions are prepared (% by weight of active material):

| | Composition A (according to the invention) | Composition B (outside the invention) |
|---|---|---|
| | | |
| Polybutene (monoolefins/isoparaffins 95/5)(MW: 2060) ⁽⁴⁾ | 10 | 0 |
| Mixture of caprylic/capric acid triglycerides ⁽¹⁾ | 21.5 | 21 |
| Denatured absolute ethyl alcohol | 2.5 | 2.5 |
| Dibutyl pentaerythrityl tetrahydroxycinnamate (2) | 0.1 | 0.1 |
| 1,2-Octanediol | 0.5 | 0.5 |
| Isopropyl palmitate ⁽³⁾ | qs 100 | qs 100 |

| | | |
|---|---|---|
| ⁽¹⁾ 70/30 C8-C10 Triglycerides (DBU MCT 7030) sold by Stéarineries Dubois ⁽²⁾ Tinogard TT sold by BASF ⁽³⁾ Isopropyl palmitate sold by BASF ⁽⁴⁾ Indopol H 1500 sold by Ineos | | |

The compositions are prepared according to the following method: the components are mixed with regular Rayneri stirring. The mixture is brought to a temperature below 50°C in order to improve and accelerate the dispersion of the polybutene.

Compositions A and B are then tested under the same conditions, in order to evaluate their cosmetic properties, after the first application, then after 10 days of application.

The sensory evaluations were carried out on two panels of men according to the following protocol:
For each formula, the tests were conducted on a panel of 60 individuals, corresponding to men 18 to 60 years old, representative of all types of facial skin, 50% of whom had sensitive skin. The individuals had a beard at least 1 cm in length, and combed their beard either with their fingers or with a comb. At least 50% of the subjects of the panel had a slightly fizzy beard.

Composition A or B was applied to the beard at least once a day, for a total period of 21 days.
The application was carried out on dry beard: the composition is placed in the palm of a hand, both hands are rubbed together, and are then applied to the beard. The beard is then combed with the fingers or with a comb.

The cosmetic properties of the beard are evaluated by the subjects at given time intervals during the study, using a 5-point value scale ("agree" - "relatively agree" - "neither agree nor do not agree" - "relatively do not agree" - "do not agree"). The values indicated in the table correspond to the proportion measured, in the panel in question, of the accumulation of "agree" and "relatively agree" responses.

| | Efficacy after the first application | | Efficacy after 10 days of application | |
|---|---|---|---|---|
| | Composition A | Composition B | Composition A | Composition B |
| Softness of the beard | 76.9% | 71.9% | 87.5% | 77.8% |
| Decrease in tackiness between the hairs | 92.3% | 87.5% | 93.8% | 85.7% |
| Decrease in frizzy appearance of the beard | 40.6% | 34.4% | 53.1% | 48.4% |
| Beard non-tacky | 92.3% | 87.5% | 92.2% | 82.5% |

The results of the tests carried out show that composition A according to the invention results in an improvement in the softness of the beard immediately after the first application, and even more significantly after 10 days of use, in comparison with a composition not containing any polybutene.

These results also show that the composition according to the invention reduces the feeling of tackiness between the hairs and of the beard as a whole, immediately after the first application and more significantly after 10 days of use.

Finally, the composition according to the invention also results in an improvement in other cosmetic properties of the beard, for instance a decrease in the frizzy appearance of the beard.

## Claims

1. Composition comprising:
a) from 8% to 15% polybutene by weight relative to the total weight of the composition;
b) at least one first non-silicone liquid fatty substance chosen from liquid monoesters of saturated or unsaturated, linear or branched, C₁-C₂₆ aliphatic monoacids and of saturated or unsaturated, linear or branched, C₁-C₂₆ aliphatic monoalcohols, the total number of carbon atoms in the esters being greater than or equal to 10; and
c) at least one second non-silicone liquid fatty substance , different from b), is chosen from natural or synthetic esters of mono-, di- or triacids with glycerol, comprising at least caprylic/capric acid triglycerides; and
wherein said composition comprises less than 5% by weight of plant oil, relative to the total weight of the composition, said composition does not contain any silicone compounds,
and **characterized in that** said composition is anhydrous.

2. Composition according to one of the preceding claims, in which said at least one first non-silicone liquid fatty substance is chosen from ethyl and isopropyl palmitates.

3. Composition according to one of the preceding claims, in which said at least one first liquid fatty substance is present in a content ranging from 10% to 80% by weight and preferably from 55% to 70% by weight, relative to the total weight of the composition.

4. Composition according to one of the preceding claims, in which said at least one second liquid fatty substance is chosen from caprylic/capric acid triglycerides.

5. Composition according to one of the preceding claims, in which said at least one second liquid fatty substance is present in a content ranging from 10% to 60% by weight and preferably from 15% to 25% by weight, relative to the total weight of the composition.

6. Composition according to one of the preceding claims, in which the composition also comprises at least one liquid polar solvent, preferably at least one alkanol and more preferably ethyl alcohol.

7. Composition according to one of the preceding claims, in which said liquid polar solvent is present in a content ranging from 2% to 20% by weight and preferably from 2.5% to 7.5% by weight, relative to the total weight of the composition.

8. Composition according to one of the preceding claims, **characterized in that** said composition does not contain any plant oils.

9. Use of a composition according to any one of the preceding claims for conditioning keratin fibres, and in particular in terms of disentangling, straightening, combability and manageability of the keratin fibres.

10. Use according to Claim 9, in which the keratin fibres are chosen from the hair, eyelashes, eyebrows, beard hairs, moustache hairs and pubic hairs, and are preferably beard and/or moustache hairs.

11. Process for conditioning keratin fibres, by treatment or application to said keratin fibres of a composition according to any one of Claims 1 to 8.

## Patentansprüche

1. Zusammensetzung, umfassend:
a) 8 bis 15 Gew.-% Polybuten, bezogen auf das Gesamtgewicht der Zusammensetzung;
b) mindestens eine erste flüssige Nichtsilikon-Fettsubstanz, die aus flüssigen Monoestern von gesättigten oder ungesättigten, linearen oder verzweigten, aliphatischen C₁-C₂₆-Monosäuren und gesättigten oder ungesättigten, linearen oder verzweigten, aliphatischen C₁-C₂₆-Monoalkoholen ausgewählt ist, wobei die Gesamtzahl der Kohlenstoffatome in den Estern größer oder gleich 10 ist; und
c) mindestens eine zweite, von b) verschiedene flüssige Nichtsilikon-Fettsubstanz, die aus natürlichen oder synthetischen Estern von Mono-, Di- oder Trisäuren mit Glycerin, die mindestens Capryl-/Caprinsäuretriglyceride umfassen, ausgewählt ist; und
wobei die Zusammensetzung weniger als 5 Gew.-% Pflanzenöl, bezogen auf das Gesamtgewicht der Zusammensetzung, umfasst und die Zusammensetzung keine Silikonverbindungen enthält,
und **dadurch gekennzeichnet, dass** die Zusammensetzung wasserfrei ist.

2. Zusammensetzung nach einem der vorhergehenden Ansprüche, wobei die mindestens eine erste flüssige Nichtsilikon-Fettsubstanz aus Ethyl- und Isopropylpalmitat ausgewählt ist.

3. Zusammensetzung nach einem der vorhergehenden Ansprüche, wobei die mindestens eine erste flüssige Fettsubstanz in einem Gehalt im Bereich von 10 bis 80 Gew.-% und vorzugsweise von 55 bis 70 Gew.-%, bezogen auf das Gesamtgewicht der Zusammensetzung, vorliegt.

4. Zusammensetzung nach einem der vorhergehenden Ansprüche, wobei die mindestens eine zweite flüssige Fettsubstanz aus Capryl-/Caprinsäuretriglyceriden ausgewählt ist.

5. Zusammensetzung nach einem der vorhergehenden Ansprüche, wobei die mindestens eine zweite flüssige Fettsubstanz in einem Gehalt im Bereich von 10 bis 60 Gew.-% und vorzugsweise von 15 bis 25 Gew.-%, bezogen auf das Gesamtgewicht der Zusammensetzung, vorliegt.

6. Zusammensetzung nach einem der vorhergehenden Ansprüche, wobei die Zusammensetzung außerdem mindestens ein flüssiges polares Lösungsmittel, vorzugsweise mindestens ein Alkanol und weiter bevorzugt Ethylalkohol umfasst.

7. Zusammensetzung nach einem der vorhergehenden Ansprüche, wobei das flüssige polare Lösungsmittel in einem Gehalt im Bereich von 2 bis 20 Gew.-% und vorzugsweise von 2,5 bis 7,5 Gew.-%, bezogen auf das Gesamtgewicht der Zusammensetzung, vorliegt.

8. Zusammensetzung nach einem der vorhergehenden Ansprüche, **dadurch gekennzeichnet, dass** sie keine Pflanzenöle enthält.

9. Verwendung einer Zusammensetzung nach einem der vorhergehenden Ansprüche zur Konditionierung von Keratinfasern, insbesondere hinsichtlich Entwirrung, Glättung, Kämmbarkeit und Handhabbarkeit der Keratinfasern.

10. Verwendung nach Anspruch 9, bei der die Keratinfasern aus dem Haar, den Wimpern, den Augenbrauen, den Barthaaren und den Schamhaaren ausgewählt sind und vorzugsweise Barthaare sind.

11. Verfahren zur Konditionierung von Keratinfasern durch Behandlung oder Aufbringen einer Zusammensetzung nach einem der Ansprüche 1 bis 8 auf die Keratinfasern.

## Revendications

1. Composition comprenant :
a) de 8 % à 15 % de polybutène en poids par rapport au poids total de la composition ;
b) au moins une première substance graisseuse liquide non de silicone choisie parmi les monoesters liquides de monoacides aliphatiques saturés ou insaturés linéaires ou ramifiés en C₁-C₂₆ et de monoalcools aliphatiques saturés ou insaturés linéaires ou ramifiés en C₁-C₂₆, le nombre total d'atomes de carbone dans les esters étant supérieur ou égal à 10 ; et
c) au moins une deuxième substance graisseuse liquide non de silicone, différente de b), choisie parmi les esters naturels ou synthétiques de mono-, di- ou triacides avec le glycérol, comprenant au moins des triglycérides d'acide caprylique/caprique ; et
dans laquelle ladite composition comprend moins de 5 % en poids d'huile végétale, par rapport au poids total de la composition, ladite composition ne contenant aucun composé de silicone,
et **caractérisée en ce que** ladite composition est anhydre.

2. Composition selon l'une des revendications précédentes, dans laquelle ladite au moins une première substance graisseuse liquide non de silicone est choisie parmi les palmitates d'éthyle et d'isopropyle.

3. Composition selon l'une des revendications précédentes, dans laquelle ladite au moins une première substance graisseuse liquide est présente en une teneur allant de 10 % à 80 % en poids, et de préférence de 55 % à 70 % en poids, par rapport au poids total de la composition.

4. Composition selon l'une des revendications précédentes, dans laquelle ladite au moins une deuxième substance graisseuse liquide est choisie parmi des triglycérides d'acide caprylique/caprique.

5. Composition selon l'une des revendications précédentes, dans laquelle ladite au moins une deuxième substance graisseuse liquide est présente en une teneur allant de 10 % à 60 % en poids, et de préférence de 15 % à 25 % en poids, par rapport au poids total de la composition.

6. Composition selon l'une des revendications précédentes, dans laquelle la composition comprend en outre au moins un solvant polaire liquide, de préférence au moins un alcanol et plus préférablement l'alcool éthylique.

7. Composition selon l'une des revendications précédentes, dans laquelle ledit solvant polaire liquide est présent en une teneur allant de 2 % à 20 % en poids, et de préférence de 2,5 % à 7,5 % en poids, par rapport au poids total de la composition.

8. Composition selon l'une des revendications précédentes, **caractérisée en ce que** ladite composition ne contient aucune huile végétale.

9. Utilisation d'une composition selon l'une quelconque des revendications précédentes pour le conditionnement de fibres kératiniques, et en particulier en termes de démêlage, de défrisage, de peignabilité et de gérabilité des fibres kératiniques.

10. Utilisation selon la revendication 9, dans laquelle les fibres kératiniques sont choisies parmi les cheveux, les cils, les sourcils, les poils de barbe, les poils de moustache et les poils pubiens, et sont de préférence des poils de barbe et/ou de moustache.

11. Procédé pour le conditionnement de fibres kératiniques, par traitement ou application sur lesdites fibres kératiniques d'une composition selon l'une quelconque des revendications 1 à 8.
